(19) Europäisches Patentamt
European Patent Office
Office européen des brevets

(11) Veröffentlichungsnummer: **0 254 183 A2**

# (12) EUROPÄISCHE PATENTANMELDUNG

(21) Anmeldenummer: 87110143.2

(51) Int. Cl.4: C07D 239/48 , A01N 43/54

(22) Anmeldetag: 14.07.87

(30) Priorität: 25.07.86 DE 3625168

(43) Veröffentlichungstag der Anmeldung: 27.01.88 Patentblatt 88/04

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Schwamborn, Michael. Dr.
von-Lohe-Strasse 9
D-5000 Koeln 80(DE)
Erfinder: Kühle, Engelbert, Dr.
von-Bodelschwingh-Strasse 42
D-5060 Bergisch-Gladbach 2(DE)
Erfinder: Santel, Hans-Joachim, Dr.
Grünstrasse 9a
D-5090 Leverkusen 1(DE)
Erfinder: Schmidt, Robert R., Dr.
Im Waldwinkel 110
D-5060 Bergisch-Gladbach 2(DE)
Erfinder: Strang, Harry, Dr.
Untendorfstrasse 6A
D-4000 Düsseldorf 31(DE)

## (54) Neue 2,4-Diaminopyrimidine.

(57) Die vorliegende Erfindung betrifft neue 2,4-Diaminopyrimidine der Formel (I)

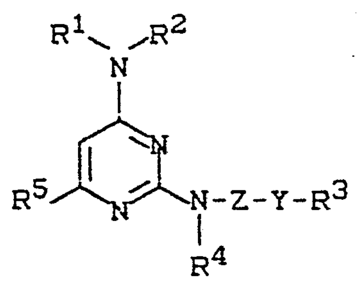

(I)

in welcher
$R^1$ und $R^4$ unabhängig voneinander für Wasserstoff oder Alkyl stehen,
$R^2$ für Wasserstoff, für gegebenenfalls durch Halogen, Cyano, Alkoxy, Alkylthio und Cyclopropyl substituiertes Alkyl, für gegebenenfalls durch Halogen substituiertes Alkenyl oder für Alkinyl steht
$R^3$ für Akyl steht,
$R^5$ für Halogen, Alkoxy oder Alkylthio steht,
Z für verzweigtes oder unverzweigtes Alkylen steht und
Y für Sauerstoff oder Schwefel steht,
mehrere Verfahren zu ihrer Herstellung und ihre Verwendung als Herbizide.

EP 0 254 183 A2

## Neue 2,4-Diaminopyrimidine

Die vorliegende Erfindung betrifft neue 2,4-Diamino-pyrimidine, Verfahren zu ihrer Herstellung sowie ihre Verwendung als Herbizide.

Es ist bereits bekannt, daß bestimmte 2,4-Diaminopyrimidine, z.B. 2-Amino-6-chlor-4-isopropylaminopyrimidin als Herbizide eingesetzt werden können (vgl. DE-OS 2 006 145). Ihre Wirkung ist unter bestimmten Bedingungen, z.B. bei niedrigen Aufwandmengen, jedoch bei verschiedenen Schadpflanzen nicht immer befriedigend. Ebenso ist ihre Verträglichkeit gegenüber bestimmten Kulturpflanzen nicht immer ausreichend.

Es wurden nun neue 2,4-Diaminopyridine der Formel (I),

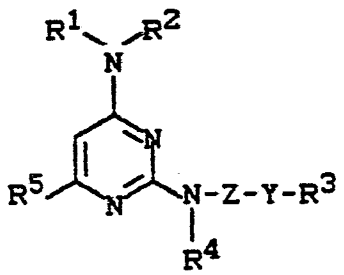

(I)

in welcher

$R^1$ und $R^4$ unabhängig voneinander für Wasserstoff oder Alkyl stehen,

$R^2$ für gegebenenfalls durch Halogen, Cyano, Alkoxy, Alkythio und Cyclopropyl substituiertes Alkyl, für gegebenenfalls durch Halogen substituiertes Alkenyl oder für Alkinyl steht,

$R^3$ für Alkyl steht,

$R^5$ für Halogen, Alkoxy oder Alkythio steht,

Z für verzweigtes oder unverzweigtes Alkylen steht und

Y für Sauerstoff oder Schwefel steht,

gefunden.

Weiterhin wurde gefunden, daß man die 2,4-Diaminopyrimidine der allgemeinen Formel (I) erhält, wenn man

A) Pyrimidine der allgemeinen Formel II,

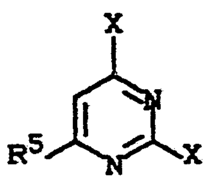

(II)

in welcher

$R^5$ die oben angegebene Bedeutung hat und

X für Halogen steht,

zunächst mit einem Amin der Formel (III)

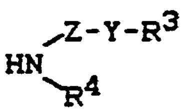

(III)

in welcher

$R^3$,$R^4$, Y und Z die oben angegebene Bedeutung haben,

gegebenenfalls in Gegenwart eines säurebindenden Mittels und gegebenenfalls in Gegenwart eines Verdünnungsmittels umsetzt zu einem Gemisch der isomeren Pyrimidinderivate der allgemeinen Formel (IVa) und (IVb) (1. Stufe)

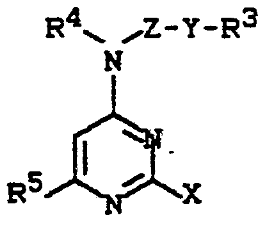 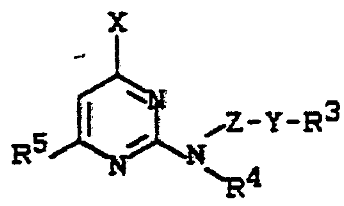

(IVa) (IVb)

in welcher

$R^3$, $R^4$, $R^5$, X, Y und Z die oben angegebene Bedeutung haben,

und anschließend nach Abtrennung der strukturisomeren Pyrimidinderivate der Formel (IVa) in einem weiteren Reaktionsschritt mit Aminen der Formel (V),

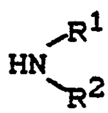

(V)

in welcher

$R^1$ und $R^2$ die oben angegebene Bedeutung haben,

gegenbenenfalls in Gegenwart eines säurebindenen Mittels und gegebenenfalls in Gegenwart eines Verdünnungsmittels zu Pyrimidinderivaten der Formel (I) umsetzt (2.Stufe),

oder wenn man

B) Pyrimidine der allgemeinen Formel (VI)

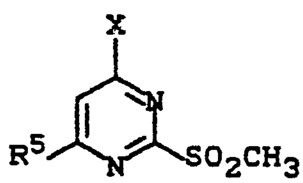

(VI)

in welcher

$R^5$ und X die oben angegebene Bedeutung haben,

zunächst mit einem Amin der Formel (III)

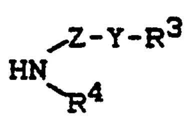

(III)

in welcher

$R^3$, $R^4$, Y und Z die oben angegebene Bedeutung haben,

gegebenenfalls in Gegenwart eines säurebindenden Mittels und gegebenenfalls in Gegenwart eines Verdünnungsmittels zu den Pyrimidinderivaten der allgemeinen Formel (IVb),

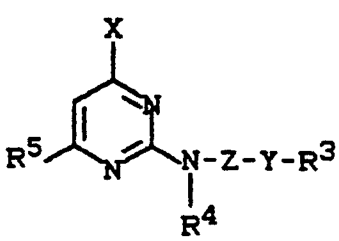

(IVb)

in welcher

$R^3$, $R^4$, $R^5$, X, Y und Z die oben angegebene Bedeutung haben,
umsetzt (1. Stufe)
und anschließend in einem weiteren Reaktionsschritt mit Aminen der Formel (V)

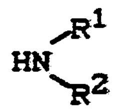 (V)

in welcher
$R^1$ und $R^2$ die oben angegebene Bedeutung haben,
gegebenenfalls in Gegenwart eines säurebindenden Mittels und gegebenenfalls in Gegenwart eines Verdünnungsmittels zu Pyrimidinderivaten der Formel (I) umsetzt (2. Stufe), oder
C) man erhält Pyrimidinderivate der Formel (I-a)

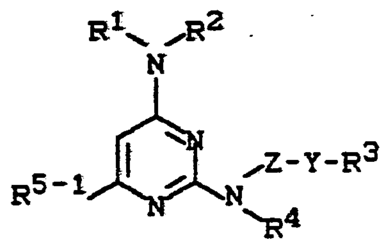 (I-a)

in welcher
$R^1$, $R^2$, $R^3$, $R^4$, Z und Y die oben angegebene Bedeutung haben und
$R^{5-1}$ für Alkythio oder Alkoxy steht,
wenn mann die Pyrimidinderivate der allgemeinen Formel (I-b)

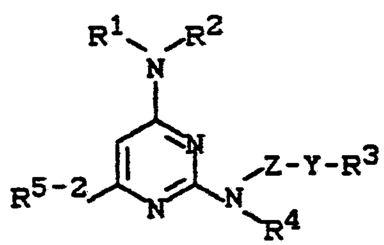 (I-b)

in welcher
$R^1$, $R^2$, $R^3$, $R^4$, Z und Y die oben angegebene Bedeutung haben und
$R^{5-2}$ für Halogen steht,
mit Alkoholen oder Mercaptanen der allgemeinen Formel (VII)
$R^6$-UH (VII)
in welcher
$R^6$ für Alkyl steht und
U für Sauerstoff oder Schwefel steht,
oder deren Alkalimetallsalze gegebenenfalls in Gegenwart eines Säurebindemittels und gegebenenfalls in Gegenwart eines Verdünnungsmittels umsetzt.

Außerdem wurde gefunden, daß die neuen 2,4-Diaminopyrimidinderivate der allgemeinen Formel (I) starke herbizide Eigenschaften aufweisen.

Die erfindungsgemäßen neuen 2,4-Diaminopyrimidinderivate der Formel (I) zeichnen sich gegenüber den vorbekannten Pyrimidinen strukturell insbesondere dadurch aus, daß die 2-Stellung einen Alkoxy-bzw. Alkylthioalkylaminosubstituenten aufweist, sowie die 4-Stellung keinen Cycloalkylaminosubstituenten aufweist.

Überraschenderweise sind die erfindungsgemäßen Wirkstoffe der Formel (I), bei besserer Kulturpflanzenverträglichkeit, deutlich wirksamer als die vorbekannten Pyrimidinderivate, wie z.B. 2-Amino-6-chlor-4-isopropylaminopyrimidin (bekannt aus DE-OS 2 006 145).

Die Kohlenstoffkette in Alkyl, Alkoxy, Alkylthio, Alkoxyalkyl, Alkylthioalkyl und Alkenyl ist jeweils geradkettig oder verzweigt. Halogen steht in den einzelnen Substituenten jeweils für Fluor, Chlor, Brom und Iod.

Von den erfindungsgemäßen 2,4-Diaminopyrimidinderivaten der Formel (I) sind bevorzugt diejenigen, in denen
$R^1$ und $R^4$ unabhängig voneinander für Wasserstoff oder Alkyl mit 1 - 6 C-Atomen stehen,
$R^2$ für gegebenenfalls einfach oder mehrfach, gleich oder verschieden durch Fluor, Chlor, Brom, Iod, Cyano und Cyclopropyl substituiertes Alkyl mit 1 -8 C-Atomen, für Alkoxyalkyl oder Alkylthioalkyl mit 1 - 6 C-Atomen je Alkylthio-bzw. Alkoxyteil und 2 - 8 C-Atomen je Alkylteil, für gegebenenfalls einfach oder mehrfach gleich oder verschieden durch Halogen substituiertes Alkenyl mit 3 - 6 C-Atomen oder für Alkinyl mit 3 - 6 C-Atomen steht,
$R^3$ für Alkyl mit 1 - 6 C-Atomen steht,
$R^5$ für Halogen, Alkoxy oder Alkylthio mit jeweils 1 -6 C-Atomen steht,
Z für eine verzweigte oder unverzweigte Alkylengruppe mit 2 - 20 C-Atomen steht und
Y für Sauerstoff oder Schwefel steht.

Aus dieser Stoffgruppe solche Verbindungen der Formel (I) besonders bevorzugt, in denen
$R^1$ und $R^4$ unabhängig voneinander für Wasserstoff oder für Alkyl mit 1 - 4 C-Atomen stehen,
$R^2$ für gegebenenfalls einfach bis dreizehnfach, gleich oder verschieden durch Fluor, Chlor, Cyano und Cyclopropyl substituiertes Alkyl mit 1 - 6 C-Atomen, für Alkoxyalkyl oder Alkylthioalkyl mit 1 - 4 C-Atomen je Alkylthio-bzw. Alkoxyteil und 2 - 6 C-Atomen je Alkylteil, für gegebenenfalls einfach bis neunfach durch Chlor substituiertes Alkenyl mit 3 - 5 C-Atomen oder für Alkinyl mit 3 - 5 C-Atomen steht,
$R^3$ für Alkyl mit 1 - 4 C-Atomen steht,
$R^5$ für Fluor, Chlor, Alkoxy oder Alkythio mit jeweils 1 - 4 C-Atomen steht,
Z für eine verzweigte oder unverzweigte Alkylengruppe mit 2 - 7 C-Atomen steht und
Y für Sauerstoff oder Schwefel steht.

Ganz besonders bevorzugt sind die Pyrimidinderivate der Formel (I) in denen $R^1$ und $R^4$ unabhängig voneinander für Wasserstoff oder Methyl stehen, und $R^2$ für Alkyl mit 1 -4 C-Atomen stehen und $R^3$, $R^5$, Z und Y die als besonders bevorzugt angegebene Bedeutung haben.

Verwendet man beispielsweise 2,4,6-Trichlorpyrimidin und 3-Methoxypropylamin als Ausgangstoffe und setzt das hierbei gebildete 4,6-Dichlor-2-(3-methoxy-propylamino)pyrimidin nach Abtrennung des isomeren 2,4-Dichlor-6-(3-methoxy-propylamino)pyrimidins mit Isopropylamin um, so kann der Reaktionsablauf für Verfahren A zusammenfassend durch das folgende Formelschema wiedergegeben werden:

**1. Stufe**

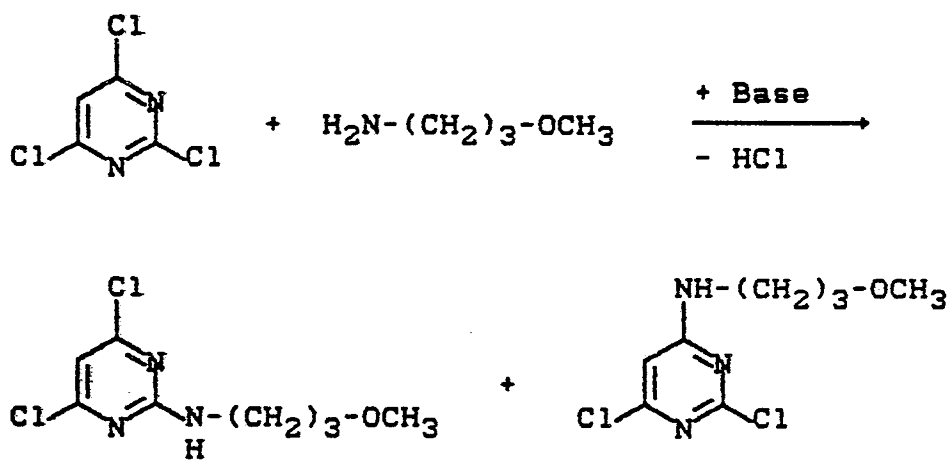


**2. Stufe**

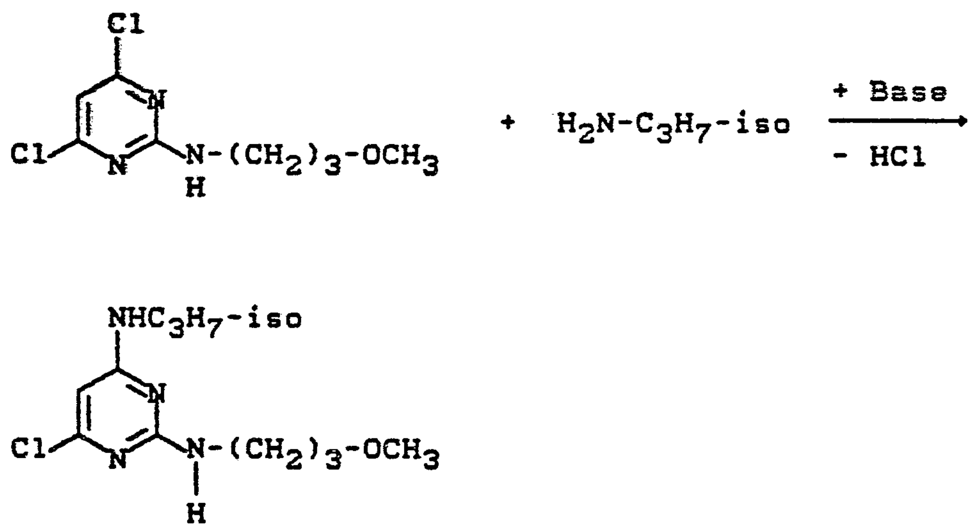


Verwendet man 4,6-Dichlor-2-methyl-sulfonyl-pyrimidin und 3-Methoxypropylamin als Ausgangsstoffe und setzt das hierbei chemoselektiv ohne isomere Nebenprodukte gebildete 4,6-Dichlor-2-(3-methoxypropylamino)-pyrimidin weiter gemäß Verfahren A um, so kann der Reaktionsablauf für Verfahren B zusammenfassend durch das folgende Formelschema wiedergegeben werden.

1.Stufe

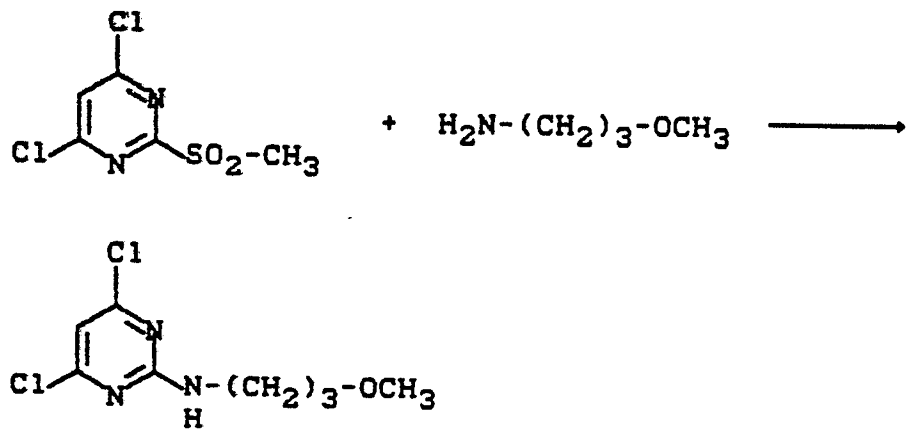

2.Stufe

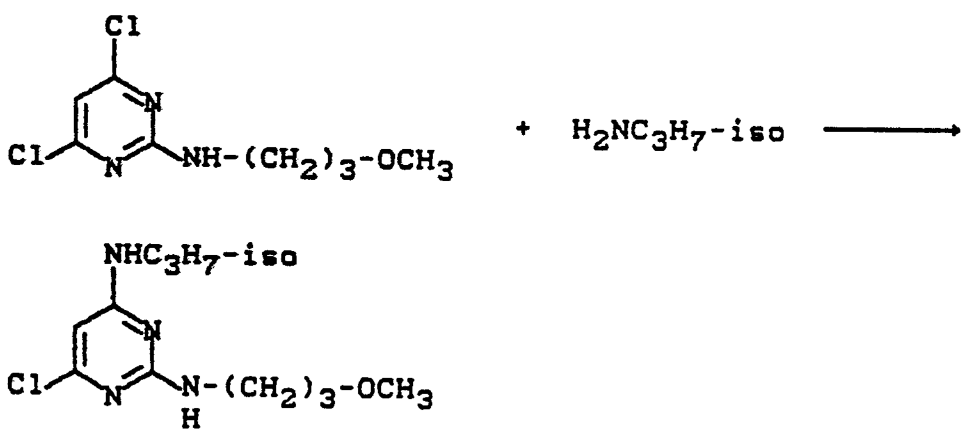

Verwendet man gemäß Verfahren C 6-Chlor-4-isopropylamino-2-(3-methoxypropylamino)pyrimidin und Natriummethanolat als Ausgangsstoffe, so kann der Reaktionsablauf zusammenfassend durch das folgende Formelschema wiedergegeben werden:

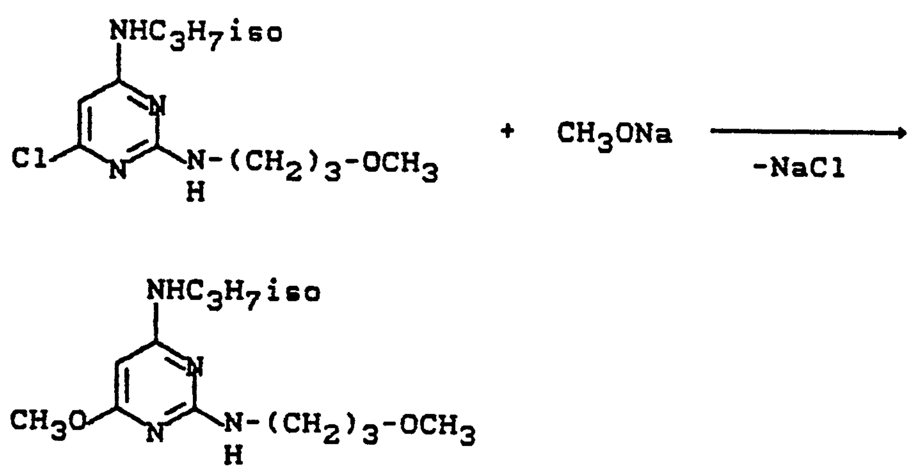

Die als Ausgangsstoffe verwendeten Pyrimidinderivate sind durch die allgemeinen Formeln (II), (VI) und (I-b) allgemein definiert. In diesen Formeln stehen $R^1$, $R^2$, $R^3$, $R^4$, $R^5$, Y und Z vorzugsweise bzw. besonders bevorzugt für diejenigen Reste, die schon bei der Beschreibung der erfindungsgemäßen Stoffe der Formel (I) als bevorzugt bzw. besonders bevorzugt für diese Substituenten genannt wurden, $R^{5-2}$ und Z stehen vorzugsweise für Chlor oder Fluor.

Die Pyrimidine der Formeln (II) und (VI) sind bekannt bzw. können nach bekannten Verfahren hergestellt werden (vgl. Ausgangsstoffe). Die Pyrimidine der Formel (I-b) sind erfindungsgemäße Verbindungen und können nach den hier beschriebenen Verfahren A und B erhalten werden.

Als Pyrimidinderivat der Formel (II) kann insbesondere 2,4,6-Trichlorpyrimidin, und als Pyrimidinderivat der Formel (VI) vorwiegend 2-Methylsulfonyl-4,6-dichlorpyrimidin eingesetzt werden.

Die weiterhin als Ausgangsstoffe verwendeten Amine sind durch die Formel (III) und (V) allgemein definiert. In diesen Formeln stehen $R^1$, $R^2$, $R^3$, $R^4$, Y und Z vorzugsweise bzw. besonders bevorzugt für diejenigen Reste, die schon bei der Beschreibung der erfindungsgemäßen Stoffe der Formel (I) als bevorzugt bzw. besonders bevorzugt für diese Substituenten genannt wurden. Die Amine der Formel (III) und (V) sind bekannt oder lassen sich nach bekannten Verfahren in analoger Weise wie die bekannten Verbindungen herstellen (vgl. z.B. Houben-Weyl, Methoden der organischen Chemie, Band XI/1, S. 548, S. 561 ff., 4. Auflage 1957; US 2 764 615).

Im einzelnen können zur Herstellung der Pyrimidinderivate der allgemeinen Formel (I) insbesondere die folgenden Amine der Formeln (III):
3-Methoxypropylamin, 3-Ethoxypropylamin, 3-n-Propoxypropylamin, 3-i-Propoxypropylamin, 3-n-Butoxy-propylamin, 3-s-Butoxypropylamin, 3-t-Butoxy-propylamin, 1-Methyl-3-methoxypropylamin, 1-Methyl-3-ethoxypropylamin, 1-Methyl-3-n-propoxypropylamin, 1-Methyl-3-i-propoxypropylamin, 1-Methyl-3-t-butoxypropylamin, 2-Methoxy ethylamin, 2-Ethoxyethylamin, 2-n-Propoxyethylamin, 2-i-Propoxyethylamin, 2-n-Butoxyethylamin, 2-s-Butoxyethylamin, 2-t-Butoxyethylamin, N-Methyl-N-3-methoxypropylamin, 3-Methylthio-propylamin
sowie die folgenden Amine der Formel (V) eingesetzt werden:
Methylamin, Ethylamin, n-Propylamin, i-Propylamin, n-Butylamin, s-Butylamin, t-Butylamin, Neopentylamin, Dimethylamin, Diethylamin, Di-n-propylamin, Di-i-propylamin, 3-Methoxypropylamin, 3-Ethoxypropylamin, 3-n-Propoxy-propylamin, 3-i-Propoxypropylamin, 3-n-Butoxypropylamin, 3-s-Butoxypropylamin, 3-t-Butoxypropylamin, 1-Methyl-3-methoxy-propylamin, 1-Mehyl-3-ethoxypropylamin, 1-Methyl-3-n-propoxypropylamin, 1-Methyl-3-i-propoxypropylamin, 1-Methyl-3-butoxypropylamin, 2-Methoxyethylamin, 2-Ethoxy-ethylamin, 2-n-Propoxyethylamin, 2-i-Propoxyethylamin, 2-n-Butoxyethylamin, 2-s-Butoxyethylamin, 2-t-Butoxyethylamin, N-Methyl-N-3-methoxypropylamin, 3-Methylthiopropylamin, Allylamin, 1-Methylallylamin, 1,1-Dimethylallylamin, Propargylamin, 1-Methylpropargylamin.

Die weiterhin als Ausgangsstoffe verwendeten Alkohole und Mercaptane sind durch die Formel (VII) allgemein definiert. In dieser Formel steht U für Sauerstoff oder Schwefel und $R^6$ bevorzugt für Alkyl mit 1-6 C-Atomen bzw. besonders bevorzugt für Alkyl mit 1 -4 C-Atomen. Die Alkohole und Mercaptane der Formel (VII) sind bekannt oder lassen sich nach bekannten Verfahren herstellen.

Im einzelnen können zur Herstellung der Pyrimidinderivate der allgemeinen Formel (I) insbesondere die folgenden Verbindungen (VII) eingesetzt werden:
Methanol, Ethanol, Propanol, i-Propanol, n-Butanol, s-Butanol, t-Butanol, Methylmercaptan, Ethylmercaptan, Propylmercaptan, t-Butylmercaptan.

Als Verdünnungsmittel für die erfindungsgemäßen Verfahren A und B kommen organische Lösungsmittel sowie Wasser in Frage. Bevorzugte organische Lösungsmittel sind Kohlenwasserstoffe wie Toluol, aliphatische Ketone wie Aceton, Methylethylketon und Diethylketon, cycloaliphatische Ether wie Tetrahydrofuran oder Dioxan, Alkohole wie Ethanol, aliphatische Ester wie Essigsäureethylester und Nitrile wie Acetonitril. Auch Gemische verschiedener organischer Lösungsmittel und Gemische von mit Wasser mischbaren organischen Lösungsmitteln mit Wasser sind als Verdünnungsmittel geeignet.

Die erfindungsgemäßen Verfahren A und B werden gegebenenfalls unter Verwendung von Säurebindemitteln durchgeführt. Als solche sind Erdalkali-und Alkalimetallhydroxide, wie Calcium-, Natrium- oder Kaliumhydroxid, ferner Ammoniak sowie tertiäre aliphatische Amine wie z.B. Triethylamin, aber auch im Überschuß eingesetztes Amin-Ausgangsprodukt der Formel (III) bzw. (VII) besonders geeignet.

Die Reaktionstemperaturen können bei den erfindungsgemäßen Verfahren A und B in einem größeren Bereich vari iert werden. Die erste Verfahrensstufe wird im allgemeinen bei Temperaturen von -80°C bis +150°C, vorzugsweise von -80°C bis +20°C durchgeführt. Die zweite Verfahrensstufe wird im allgemeinen bei Temperaturen von -80°C bis +250°C, vorzugsweise von -20°C bis +150°C durchgeführt.

Die Umsetzung wird im Druckbereich von 1 bis etwa 10 bar durchgeführt.

Bei der Durchführung der erfindungsgemäßen Verfahren A und B setzt man auf 1 Mol Pyrimidin der Formel (II) bzw. (VI) in der ersten Stufe im allgemeinen 1 bis 2 Mol, vorzugsweise 1 bis 1,1 Mol Amin der Formel (III) und gegebenenfalls 1 bis 2 Mol, vorzugsweise 1 bis 1,2 Mol Säurebinder ein, wobei als Säurebinder das Amin der Formel (II) verwendet werden kann. Besonders bevorzugt wird unter Einsatz stöchiometrischer Molverhältnisse gearbeitet. Für die zweite Verfahrensstufe gilt Entsprechendes.

Gegebenenfalls in der ersten Stufe des Verfahrens A entstandene strukturisomere Pyrimidinnebenprodukte der Formel (IVa) können in einfacher Weise nach bekannten Methoden abgetrennt werden, insbesondere durch Umkristallisation, Chromatographie oder Wasserdampfdestillation (vgl. z.B. DE-OS 2 006 145), so daß sich die Pyrimidinderivate der Formel (IVb) in ausreichend reiner Form isolieren lassen.

Als Verdünnungsmittel für das erfindungsgemäße Verfahren C kommen organische Lösungsmittel in Frage.

Bevorzugte organische Losungsmittel sind Alkohole wie Ethanol, Ketone wie Diethylketon, cycloaliphatische Ether wie Tetrahydrofuran oder Dioxan sowie Kohlenwasserstoffe wie Toluol.

Das erfindungsgemäße Verfahren C wird gegebenenfalls unter Verwendung von Säurebindemitteln durchgeführt. Als solche sind tertiäre aliphatische Amine wie z.B. Triethylamin oder Alkalimetallhydride wie z.B. Natriumhydrid besonders geeignet.

Die Reaktionstemperaturen können bei dem erfindungsgemäßen Verfahren C in einem größeren Bereich variiert werden. Im allgemeinen wird das Verfahren bei 0°C bis +250°C, vorzugsweise bei +50°C bis +180°C durchgeführt.

Bei der Durchführung des erfindungsgemäßen Verfahrens C setzt man auf 1 Mol Pyrimidin der allgemeinen Formel (I-b) 1 bis 20 Mol, vorzugsweise 1 - 10 Mol der Verbindung der Formel (VII) und 1 bis 20 Mol, vorzugsweise 1 - 10 Mol, Säurebindemittel ein.

Zur Durchführung des erfindungsgemäßen Verfahrens C setzt man die Verbindungen der Formel (VII) vorteilhaft in Form ihrer Alkalimetallsalze ein.

Die erfindungsgemäßen Wirkstoffe können als Defoliants, Desiccants, Krautabtötungsmittel und insbesondere als Unkrautvernichtungsmittel verwendet werden. Unter Un kraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Ledpidium, Galium, Stellaria, Matricaria, Anthemis, Galinsoga, Chenopodium, Urtica, Senecio, Amaranthus, Portulaca, Xanthium, Convolvulus, Ipomcea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Die erfindungsgemäßen Wirkstoffe eigenen sich insbesondere im Nachauflaufverfahren zur selektiven Bekämpfung mono-und dikotyler Unkräuter, insbesondere in monokotylen Kulturen.

Bei der Anwendung im Nachauflaufverfahren können die erfindungsgemäßen Verbindungen allein oder in Kombination mit oberflächenaktiven Substanzen, emulgierbaren Ölen und anderen Additiven ausgebracht werden.

Die Wirkstoffe können in die üblichen Formulierungen überführt werden, wie Lösungen, Emulsionen, Spritzpul ver, Suspensionen, Pulver, Stäubemittel, Pasten, lösliche Pulver, Granulate, Suspensionens-Emulsions-Konzentrate, wirkstoffimprägnierte Natur-und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder - schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen in wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polar Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Ge steinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sipiolith, Dolomit sowie synthetische Granulate aus anorganischen und organischen Mehlen sowie Granulate aus organischen Material wie Sägemehl, Kokosnußschalen, Maiskolben und Tabakstengeln; als Emulgier-und/oder schaumerzeugende Mittel kommen in Frage: z.B. nichtionogine und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewischtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.

Als Mischpartner kommen auch Harnstoffe (z.B. Methabenzthiazuron, Chlortoluron, Isoproturon), Sulfonylharnstoffe (z.B. Chlorsulfuron, Methsulfuron-methyl), Triazine (z.B. Atrazin, Cyanazin, Terbutryne), Triazinone (z.B. Metribuzin, Ethiozin), Triazindione (z.B. Amethydione), Phenoxyalkancarbonsäuren (z.B. 2,4D; 2,4DP; MCPA; MCPP), Benzonitrile (z.B. Ioxynil, Bromoxynil, Pyridyloxyessigsäuren (z.B. Fluroxypyr), Imidazolinone (z.B. Imazamethabenz), Aryloxy-oder Heteroaryloxyphenoxypropionsäuren (z.B. Illoxan, (2R)-2-[4-(3,5-Dichlorpyrid-2-yloxy)-phenoxy]-propionsäure-trimethyl silylmethylester), Bentazone und Pyridate in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmangen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1 (Verfahren A)

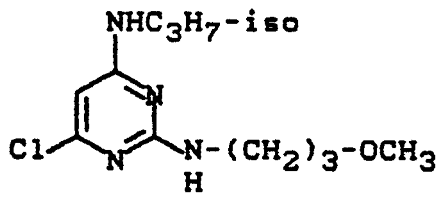

6-Chloro-4-isopropylamino-2-(3-methoxypropylamino)-pyrimidin

a) 4,6-Dichloro-2-(3)-methoxypropylamino)-pyrimidin (1. Stufe)

Zu 35 g (0.19 mol) 2,4,6-Trichlorpyrimidin in 150 ml Ethanol tropft man bei 25°C 37,4 g (0,42 mol) 3-Methoxypropylamino und rührt eine Stunde nach. Das Reaktionsgemisch wird mit Wasser versetzt und mit Dichlormethan extrahiert. Die Dichlormethanphase wird eingeengt und der resultierende Rückstand wird einer Wasserdampfdestillation unterzogen, wobei 5 g (11 % der Theorie) des gewünschten Pyrimidins überdestillieren. Fp. 53°C.

b) 6-Chlor-4-isopropylamino-2-(3-methoxypropylamino)-pyrimidin (2. Stufe)

5 g (0,021 mol) 4,6-Dichlor-2-(3-methoxypropylamino)-pyrimidin werden in 100 ml Dioxan gelöst. Hierzu tropft man 3,6 g (0,063 mol) wasserfreies Isopropylamin und erhitzt 3 Stunden zum Rückfluß. Das Reaktionsgemisch wird in 500 ml Eiswasser eingerührt. Nach Absaugen und Trocknen erhält man 4 g (74 % d. Theorie) des gewünschten Pyrimidins. Fp. 125°C.

Beispiel 1 (Verfahren B)

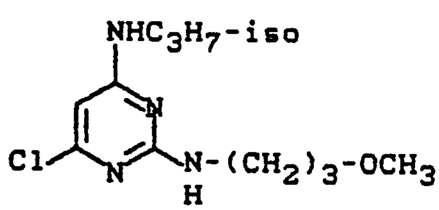

6-Chlor-4-isopropylamino-2-(3-methoxypropylamino)-pyrimidin

a) 4,6-Dichlor-2-(3-methoxypropylamino)pyrimidin (1. Stufe)

Zu 195 g (0,86 mol) 4,6-Dichlor-2-methylsulfonylpyrimidin in 2 l Essigsäureethylester tropft man bei -78°C 153 g (1,72 mol) 3-Methoxypropylamino und läßt das Reaktionsgemisch über 16 Stunden auf 25°C auftauen. Die organische Phase wird mit 2 l Wasser gewaschen , getrocknet und eingeengt. Man erhält 176 g (89 % d. Theorie) des gewünschten Pyrimidins. Fp. 53°C.

b) 6-Chlor-4-isopropylamino-2-(3-methoxypropylamino-pyrimidin (2. Stufe)

Hier verfährt man wie bei Verfahren A

Beispiel 2 (Verfahren C)

4-Isopropylamino-6-methoxy-2-(3-methoxypropylamino)-pyrimidin

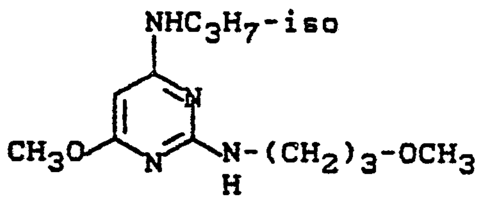

5 g (0,2 mol) 6-Chlor-4-isopropylamino-2-(3-methoxypropylamino)-pyrimidin werden in 200 ml Dioxan gelöst und unter Zugabe von 14 g (0,25 mol) Natriummethanolat im Autoklaven 12 h auf 160°C erhitzt. Nach Abdestillieren des Solvens wird der verbleibende Rückstand in Dichlormethan aufgenommen und die organische Phase fünfmal mit je 100 ml Wasser gewaschen. Nach Trocknen über Natriumsulfat erhält man 2,6 g(54 % der Theorie) des gewünschten Pyrimidins. $n_D^{20}$ : 1,5330

In analoger Weise zu Beispiel 1 und 2 und den vorne beschriebenen Verfahren erhält man die in der nachfolgenden Tabelle 1 genannten Pyrimidinderivate der allgemeinen Formel (I)

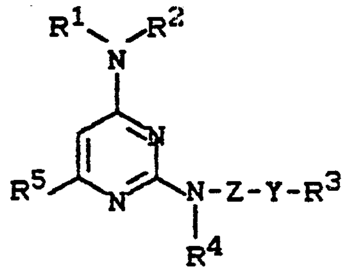

**Tabelle 1**

| Beispiel-Nr. | $R^1$ | $R^2$ | $R^3$ | $R^4$ | $R^5$ | Y | Z | Schmelzpunkt/°C $n_D$ |
|---|---|---|---|---|---|---|---|---|
| 3 | H | $i-C_3H_7$ | $CH_3$ | H | $SCH_3$ | O | $-(CH_2)_3-$ | 1.5614 |
| 4 | H | $CH_3$ | $CH_3$ | H | Cl | O | $-(CH_2)_3-$ | 68 |
| 5 | H | $C_2H_5$ | $CH_3$ | H | Cl | O | $-(CH_2)_3$ | 94 |
| 6 | $CH_3$ | $CH_3$ | $CH_3$ | H | Cl | O | $-(CH_2)_3-$ | 89 |
| 7 | H | $C_2H_5$ | $CH_3$ | H | Cl | S | $-(CH_2)_2-$ | 78 |
| 8 | H | $i-C_3H_7$ | $CH_3$ | H | Cl | S | $-(CH_2)_2-$ | 68 |
| 9 | H | $C_2H_5$ | $C_2H_5$ | H | Cl | S | $-(CH_2)_2-$ | 1.5900 |
| 10 | H | $i-C_3H_7$ | $C_2H_5$ | H | Cl | S | $-(CH_2)_2-$ | 65 |

**Tabelle 1 (Fortsetzung)**

| Beispiel-Nr. | $R^1$ | $R^2$ | $R^3$ | $R^4$ | $R^5$ | Y | Z | Schmelzpunkt/°C $n_D$ |
|---|---|---|---|---|---|---|---|---|
| 11 | H | $C_2H_5$ | $CH_2$-i-$C_3H_7$ | H | Cl | O | $-(CH_2)_3-$ | 1.5348 |
| 12 | H | i-$C_3H_7$ | $CH_2$-i-$C_3H_7$ | H | Cl | O | $-(CH_2)_3-$ | 52 |
| 13 | H | $C_2H_5$ | $C_2H_5$ | H | Cl | O | $-(CH_2)_2-$ | 1.5560 |
| 14 | H | i-$C_3H_7$ | $C_2H_5$ | H | Cl | O | $-(CH_2)_2-$ | 1.5470 |
| 15 | H | $C_2H_5$ | i-$C_3H_7$ | H | Cl | O | $-(CH_2)_3-$ | 67 |
| 16 | H | i-$C_3H_7$ | i-$C_3H_7$ | H | Cl | O | $-(CH_2)_3-$ | 82 |
| 17 | H | $C_2H_5$ | $CH_3$ | H | Cl | O | $-(CH_2)_3-CH(CH_3)-$ | 55 |

Tabelle 1 (Fortsetzung)

| Beispiel-Nr. | $R^1$ | $R^2$ | $R^3$ | $R^4$ | $R^5$ | Y | Z | Schmelzpunkt/°C $n_D$ |
|---|---|---|---|---|---|---|---|---|
| 18 | H | $i-C_3H_7$ | $CH_3$ | H | Cl | O | $-(CH_2)_3-CH(CH_3)-$ | 82 |
| 19 | H | $C_2H_5$ | $n-C_5H_{11}$ | H | Cl | O | $-(CH_2)_3-$ | 44 |
| 20 | H | $i-C_3H_7$ | $n-C_5H_{11}$ | H | Cl | O | $-(CH_2)_3-$ | 1.5290 |
| 21 | H | $C_2H_5$ | $CH_3$ | H | Cl | O | $-(CH_2)_2-$ | 98 |
| 22 | H | $i-C_3H_7$ | $CH_3$ | H | Cl | O | $-(CH_2)_2-$ | 94 |
| 23 | H | $C_2H_5$ | $n-C_3H_7$ | H | Cl | O | $-(CH_2)_2-$ | 76 |
| 24 | H | $C_2H_5$ | $C_2H_5$ | H | Cl | O | $-(CH_2)_3-$ | 70 |
| 25 | H | $i-C_3H_7$ | $C_2H_5$ | H | Cl | O | $-(CH_2)_3-$ | 100 |

**Tabelle 1** (Fortsetzung)

| Beispiel-Nr. | $R^1$ | $R^2$ | $R^3$ | $R^4$ | $R^5$ | Y | Z | Schmelzpunkt/°C $n_D$ |
|---|---|---|---|---|---|---|---|---|
| 26 | H | $C_2H_5$ | $CH_3$ | H | Cl | O | $-CH_2-CH(CH_3)-CH_2-$ | 77 |
| 27 | H | $i-C_3H_7$ | $CH_3$ | H | Cl | O | $-CH_2-CH(CH_3)-CH_2-$ | 95 |
| 28 | H | $C_2H_5$ | $C_2H_5$ | H | Cl | O | $-CH(CH_3)-(CH_2)_2-$ | 1.5455 |
| 29 | H | $i-C_3H_7$ | $C_2H_5$ | H | Cl | O | $-CH(CH_3)-(CH_2)_2-$ | 40 |

**Tabelle 1** (Fortsetzung)

| Beispiel-Nr. | $R^1$ | $R^2$ | $R^3$ | $R^4$ | $R^5$ | Y | Z | Schmelzpunkt/°C $n_D$ |
|---|---|---|---|---|---|---|---|---|
| 30 | H | $C_2H_5$ | $C_2H_5$ | H | Cl | O | $-CH_2-CH-CH_2-$<br>$CH_3$ | 73 |
| 31 | H | $i-C_3H_7$ | $C_2H_5$ | H | Cl | O | $-CH_2-CH-CH_2-$<br>$CH_3$ | 94 |
| 32 | H | $C_2H_5$ | $C_2H_5$ | H | Cl | O | $-(CH_2)_2-CH-$<br>$CH_3$ | 1.5415 |
| 33 | H | $C_2H_5$ | $CH_3$ | H | Cl | O | $-(CH_2)_2-CH-$<br>$CH_3$ | 55 |
| 34 | H | $i-C_3H_7$ | $CH_3$ | H | Cl | O | $-(CH_2)_2-CH-$<br>$CH_3$ | 1.5145 |

Ausgangsstoffe

Die als Ausgangsstoffe verwendeten Pyrimidine der Formel (II-1) und (VI-1) sind bekannt.

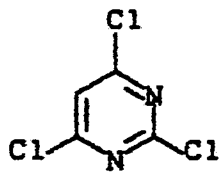

(II-1): bekannt aus:
Baddiley u.Topham
J. Chem. Soc. 1944, 678

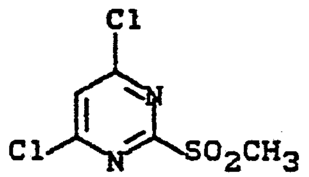

(IV-1): bekannt aus:
J. M. Spragne u.
T. B. Johnson
J. Amer. Chem. Soc. 57, 2252 (1935).

Anwendungsbeispiele

In dem folgenden Anwendungsbeispiel wird die nachstehend aufgeführte Verbindung als Vergleichssubstanz herangezogen:

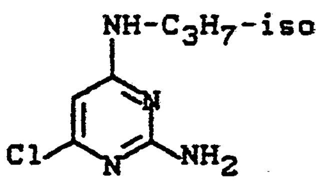

(A)

bekannt aus DE-OS 2 006 145.

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

In diesem Test zeigt z.B. die Verbindung gemäß Herstellungsbeispiel (1) eine bessere Nutzpflanzenverträglichkeit, insbesondere in Weizen, und eine deutlich bessere herbizide Wirkung gegen Unkräuter wie beispielsweise Galium, Galinsoga, Helianthus, und Matricaria als die Vergleichssubstanz (A).

**Ansprüche**

1. 2,4-Diaminopyrimidine der Formel (I)

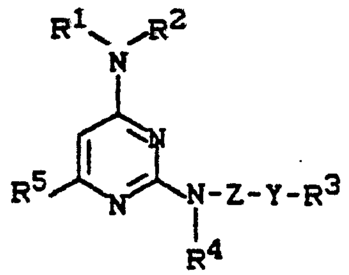

(I)

in welcher
$R^1$ und $R^4$ unabhängig voneinander für Wasserstoff oder Alkyl stehen,
$R^2$ für gegebenenfalls durch Halogen, Cyano, Alkoxy, Alkylthio und Cyclopropyl substituiertes Alkyl, für gegebenenfalls durch Halogen substituiertes Alkenyl oder für Alkinyl steht,
$R^3$ für Alkyl steht,
$R^5$ für Halogen, Alkoxy oder Alkylthio steht,
Z für verzweigtes oder unverzweigtes Alkylen steht und
Y für Sauerstoff oder Schwefel steht.

2. 2,4-Diaminopyrimidine der Formel (I) gemäß Anspruch 1, in welcher
$R^1$ und $R^4$ unabhängig voneinander für Wasserstoff oder Alkyl mit 1 - 6 C-Atomen stehen,
$R^2$ für gegebenenfalls einfach oder mehrfach, gleich oder verschieden durch Fluor, Chlor, Brom, Iod, Cyano und Cyclopropyl substituiertes Alkyl mit 1 - 8 C-Atomen, für Alkoxyalkyl oder Alkylthioalkyl mit 1-6 C-Atomen je Alkylthio-bzw. Alkoxyteil und 2 -8 C-Atomen je Alkylteil, für gegebenenfalls einfach oder mehrfach gleich oder verschieden durch Halogen substituiertes Alkenyl mit 3 -6 C-Atomen oder für Alkinyl mit 3 -6 C-Atomen steht,
$R^3$ für Alkyl mit 1 - 6 C-Atomen steht,
$R^5$ für Halogen, Alkoxy oder Alkylthio mit jeweils 1 -6 C-Atomen steht,
Z für eine verzweigte oder unverzweigte Alkylengruppe mit 2 - 10 C-Atomen steht und
Y für Sauerstoff oder Schwefel steht.

3. 2,4-Diaminopyrimidine der Formel (I) gemäß Anspruch 1, in welcher
$R^1$ und $R^4$ unabhängig voneinander für Wasserstoff oder für Alkyl mit 1 - 4 C-Atomen stehen,
$R^2$ für gegebenenfalls einfach bis dreizehnfach, gleich oder verschieden durch Fluor, Chlor, Cyano und Cyclopropyl substituiertes Alkyl mit 1 - 6 C-Atomen, für Alkoxyalkyl oder Alkylthioalkyl mit 1 - 4 C-Atomen je Alkylthio-bzw. Alkoxyteil und 2 -6 C-Atomen je Alkylteil, für gegebenenfalls einfach bis neunfach durch Chlor substituiertes Alkenyl mit 3 - 5 C-Atomen oder für Alkinyl mit 3 -5 C-Atomen steht,
$R^3$ für Alkyl mit 1 - 4 C-Atomen steht,
$R^5$ für Fluor, Chlor, Alkoxy oder Alkylthio mit jeweils 1 - 4 C-Atomen steht,
Z für eine verzweigte oder unverzweigte Alkylengruppe mit 2 - 7 C-Atomen steht und
Y für Sauerstoff oder Schwefel steht.

4. Verfahren zur Herstellung von 2,4-Diaminopyrimidinen der Formel (I),

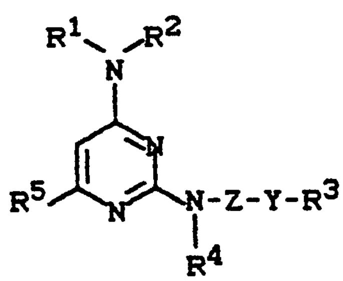

(I)

in welcher
$R^1$ und $R^4$ unabhängig voneinander für Wasserstoff oder Alkyl stehen,
$R^2$ für gegebenenfalls durch Halogen, Cyano, Alkoxy, Alkylthio und Cyclopropyl substituiertes Alkyl, für gegebenenfalls durch Halogen substituiertes Alkenyl oder für Alkinyl steht
$R^3$ für Alkyl steht,

$R^5$ für Halogen, Alkoxy oder Alkylthio steht,
Z für verzweigtes oder unverzweigtes Alkylen steht und
Y für Sauerstoff oder Schwefel steht,
dadurch gekennzeichnet, daß man
A) Pyrimidine der allgemeinen Formel (II),

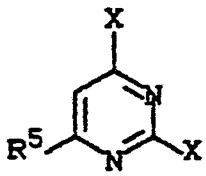

(II)

in welcher
$R^5$ die oben angegebene Bedeutung hat und
X für Halogen steht,
zunächst mit einem Amin der Formel (III)

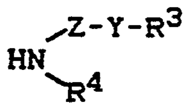

(III)

in welcher
$R^3$, $R^4$, Y und Z die oben angegebene Bedeutung haben,
gegebenenfalls in Gegenwart eines säurebindenden Mittels und gegebenenfalls in Gegenwart eines Verdünnungsmittels umsetzt zu einem Gemisch der isomeren Pyrimidinderivate der allgemeinen Formel (IVa) und (IVb) (1. Stufe)

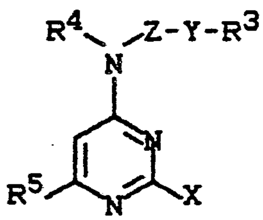

(IVa)

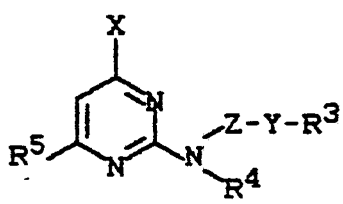

(IVb)

in welcher
$R^3$, $R^4$, $R^5$, X, Y und Z die oben angegebene Bedeutung haben,
und anschließend nach Abtrennung der strukturisomeren Pyrimidinderivate der Formel (IVa) in einem weiteren Reaktionsschritt mit Aminen der Formel (V),

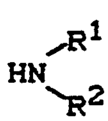

(V)

in welcher
$R^1$ und $R^2$ die oben angegebene Bedeutung haben,
gegebenenfalls in Gegenwart eines säurebindenen Mittels und gegebenenfalls in Gegenwart eines Verdünnungsmittels zu Pyrimidinderivaten der Formel (I) umsetzt (2.Stufe),
oder daß man
B) Pyrimidine der allgemeinen Formel (VI)

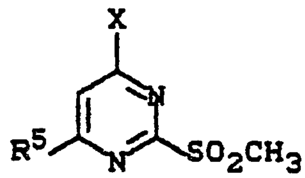

(VI)

in welcher
$R^5$ und X die oben angegebene Bedeutung haben, zunächst mit einem Amin der Formel (III)

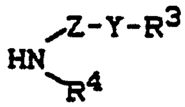

(III)

in welcher
$R^3$, $R^4$, Y und Z die oben angegebene Bedeutung haben, gegebenenfalls in Gegenwart eines säurebindenden Mittels und gegebenenfalls in Gegenwart eines Verdünnungsmittels zu den Pyrimidinderivaten der allgemeinen Formel (IVb),

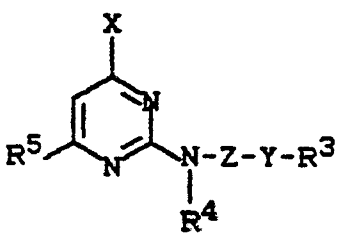

(IVb)

in welcher
$R^3$, $R^4$, $R^5$, X, Y und Z die oben angegebene Bedeutung haben, umsetzt (1. Stufe)
und anschließend in einem weiteren Reaktionsschritt mit Aminen der Formel (V)

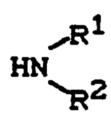

(V)

in welcher
$R^1$ und $R^2$ die oben angegebene Bedeutung haben, gegebenenfalls in Gegenwart eines säurebindenden Mittels und gegebenenfalls in Gegenwart eines Verdünnungsmittels zu Pyrimidinderivaten der Formel (I) umsetzt (2. Stufe), oder daß man
C) Pyrimidinderivate der Formel (I-a) erhält,

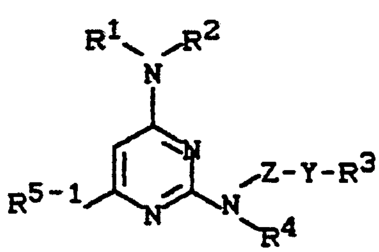

(I-a)

in welcher
$R^1$, $R^2$, $R^3$, $R^4$, Z und Y die oben angegebene Bedeutung haben und
$R^{5-1}$ für Alkylthio oder Alkoxy steht,
wenn man die Pyrimidinderivate der allgemeinen Formel (I-b)

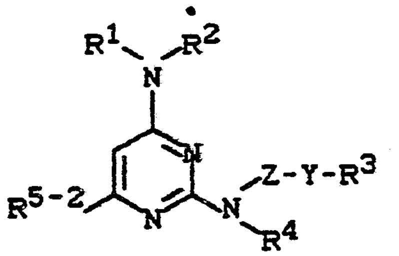

(I-b)

in welcher
$R^1$, $R^2$, $R^3$, $R^4$, Z und Y die oben angegebene Bedeutung haben und
$R^{5-2}$ für Halogen steht,
mit Alkoholen oder Mercaptanen der allgemeinen Formel (VII)
$R^6$-UH (VII)
in welcher
$R^6$ für Alkyl steht und
U für Sauerstoff oder Schwefel steht,
oder deren Alkalimetallsalze gegebenenfalls in Gegenwart eines Säurebindemittels und gegebenenfalls in Gegenwart eines Verdünnungsmittels umsetzt.

5. Herbizide Mittel, gekennzeichnet, durch einen Gehalt an mindestens einem 2,4-Diaminopyrimidin der Formel (I) gemäß den Ansprüchen 1 bis 4.

6. Verfahren zur Bekämpfung von Unkräutern, dadurch gekennzeichnet, daß man 2,4-Diaminopyrimidine der Formel (I) gemäß den Ansprüchen 1 bis 4 auf die Unkräuter und/oder ihren Lebensraum einwirken läßt.

7. Verwendung von 2,4-Diaminopyrimidinen der Formel (I) gemäß den Ansprüchen 1 bis 4 zur Bekämpfung von Unkräutern.

8. Verfahren zur Herstellung von herbiziden Mitteln, dadurch gekennzeichnet, daß man 2,4-Diaminopyrimidine der Formel (I) gemäß den Ansprüchen 1 bis 4 mit Streckmitteln und/oder oberflächenaktiven Substanzen vermischt.